(19) Europäisches Patentamt / European Patent Office / Office européen des brevets

(11) EP 3 565 518 B1

(12) # EUROPEAN PATENT SPECIFICATION

(45) Date of publication and mention of the grant of the patent:
**14.08.2024 Bulletin 2024/33**

(21) Application number: **16925778.9**

(22) Date of filing: **28.12.2016**

(51) International Patent Classification (IPC):
***A61K 8/02*** *(2006.01)* ***A61K 8/92*** *(2006.01)*
***A61K 8/85*** *(2006.01)* ***A61K 8/37*** *(2006.01)*
***A61Q 1/04*** *(2006.01)* ***A61Q 1/08*** *(2006.01)*
***A61K 8/36*** *(2006.01)* ***A61K 8/39*** *(2006.01)*
***A61Q 1/06*** *(2006.01)*

(52) Cooperative Patent Classification (CPC):
**A61K 8/0216; A61K 8/361; A61K 8/37; A61K 8/375; A61K 8/39; A61K 8/85; A61K 8/922; A61Q 1/06;** A61K 2800/31; A61Q 1/08

(86) International application number:
**PCT/CN2016/112682**

(87) International publication number:
**WO 2018/119762 (05.07.2018 Gazette 2018/27)**

(54) **SOLID ANHYDROUS COMPOSITION FOR CARING FOR AND/OR MAKING UP KERATIN MATERIALS**

FESTE WASSERFREIE ZUSAMMENSETZUNG ZUR PFLEGE UND/ODER AUFBEREITUNG VON KERATINMATERIALIEN

COMPOSITION ANHYDRE SOLIDE POUR LE SOIN ET/OU LE MAQUILLAGE DE MATIÈRES KÉRATINIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**13.11.2019 Bulletin 2019/46**

(73) Proprietors:
- **L'OREAL**
  **75008 Paris (FR)**
  Designated Contracting States:
  **AT BE BG CH CY CZ DE DK EE ES FI FR GB GR HR HU IE IS IT LI LT LU LV MC MK MT NL NO PL PT RO RS SE SI SK SM TR**
- **Xu, Zhen**
  **Wanchai**
  **Hong Kong (CN)**
  Designated Contracting States:
  **AL**

(72) Inventor: **XU, Zhen**
**Shanghai 201206 (CN)**

(74) Representative: **Casalonga**
**Casalonga & Partners**
**Bayerstraße 71/73**
**80335 München (DE)**

(56) References cited:
**WO-A1-2009/147089**
**WO-A2-2007/066309**
**CN-A- 101 472 594**
**JP-A- 2007 210 958**
**US-A1- 2005 026 795**
**US-A1- 2005 287 101**
**US-A1- 2007 134 181**

**EP 3 565 518 B1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The present invention relates to a composition for caring for and/or making up keratin materials such as the skin and the lips, preferably the lips. More particularly, the present invention relates to a solid anhydrous composition with desired hardness and leaving a non-sticky feeling upon application onto keratin materials. The present invention also relates to a process for caring for and/or making up keratin materials such as the skin and the lips, preferably the lips. The reference to invention throughout this description is to be solely understood as reference to the invention as defined by the appended claims.

### BACKGROUND

Compositions for caring for and/or making up the skin and/or the lips are produced to satisfy the need of moisturizing or hydration of the skin and the lips.

These compositions in the form of a stick, must, on the one hand, satisfy mechanical requirements in order to ensure the glideness and wear properties of the stick during application and to prevent it from breaking, and, on the other hand, satisfy transfer qualities so as to ensure comfortable application and also a sufficient and good-quality deposit on the lips.

On the other hand, natural ingredients, i.e., ingredients of natural origin, are of great interest to the consumers. Many oils or pasty compounds with natural origin are used in formulating compositions for caring for and/or making up of skin or lips.

Efforts had been made to formulate compositions comprising natural ingredients, which present good properties as mentioned above.

However, it is difficult to control the hardness of this type of products. A lipstick made of natural ingredients (mainly triglycerids and esters) are often either too hard, which is difficult to apply, or too soft, which is easy to lapse in a container.

In addition, some lipsticks will leave a sticky film on the lips upon application.

The search consequently continues for compositions with desired hardness and leaving a non-sticky feeling upon application onto keratin materials.

### SUMMARY OF THE INVENTION

The objective of the present invention is thus to overcome the problems described above and to propose a composition with desired hardness and leaving a non-sticky feeling upon application onto keratin materials.

The above objective is achieved by the present invention, as defined in claim 1 as appended.

It has been found that with the combination of a) greater than or equal to 5% by weight in accordance to the total weight of the composition, of at least one oil chosen from fatty acid triglycerides, b) at least one pasty compound chosen from an ester of dimer dilinoleic acid and polyol(s) or an ester thereof, and c) at least one wax of solid linear ester derived from $C_6$-$C_{30}$ fatty acid, the solid anhydrous composition will have desired hardness and result in non-sticky feeling upon application onto keratin materials.

In addition, the solid anhydrous composition according to the present invention is easy to apply, i.e. it has good spreadability and pay-off, and would not lapse in a container, i.e., it has good shape stability.

Furthermore, the solid anhydrous composition according to the present invention has homogeneous wear after application.

### DETAILD DESCRIPTION OF THE INVENTION

The solid anhydrous composition according to the present invention is as defined in the appended claims.

According to a preferred embodiment, all of a) at least one oil chosen from fatty acid triglycerides, b) at least one pasty compound chosen from an ester of dimer dilinoleic acid and polyol(s) or an ester thereof and c) are of plant origin.

The term "solid" used herein means the hardness of the composition at 20°C and at atmospheric pressure (760 mmHg) is greater than or equal to 30 $Nm^{-1}$ when it is measured according to the protocol described below.

The composition whose hardness is to be determined is stored at 20°C for 24 hours before measuring the hardness.

The hardness may be measured at 20°C via the "cheese wire" method, which consists in transversely cutting a wand of product, which is preferably a circular cylinder, by means of a rigid tungsten wire 250 μm in diameter, by moving the wire relative to the stick at a speed of 100 mm/minute.

The hardness of the samples of compositions of the present invention, expressed in $Nm^{-1}$, is measured using a DFGS2 tensile testing machine from the company Indelco-Chatillon.

The measurement is repeated three times and then averaged. The average of the three values read using the tensile testing machine mentioned above, noted Y, is given in grams. This average is converted into Newtons and then divided by L which represents the longest distance through which the wire passes. In the case of a cylindrical wand, L is equal to the diameter (in metres).

The hardness is converted into $Nm^{-1}$ by the equation below:

$$(Y \times 10^{-3} \times 9.8)/L$$

For a measurement at a different temperature, the composition is stored for 24 hours at this new temperature before the measurement.

According to this measuring method, the composition according to the present invention preferably has hardness at 20°C and at atmospheric pressure of greater than or equal to 40 $Nm^{-1}$ and preferably greater than 50 $Nm^{-1}$.

Preferably, the composition according to the present invention especially has a hardness at 20°C of less than 500 $Nm^{-1}$, especially less than 400 $Nm^{-1}$ and preferably less than 300 $Nm^{-1}$.

Advantageously, these compositions have a shear value ranging from 75 to 150 and preferably from 100 to 125 gF. Thus, these compositions may be formulated in standard packaging that does not require any composition support means.

For the purposes of the present invention, the term "anhydrous" means that the composition according to the present invention contains less than 2% and preferably less than 0.5% by weight of water relative to the total weight of the composition. Where appropriate, such small amounts of water may be provided by ingredients of the composition that contain it in residual amount, but are not deliberately provided.

Preferably, the "keratin material" according to the present invention is the skin and the lips. By "skin", we intend to mean all the body skin, including the scalp. Still preferably, the keratin material is the lips.

**Oil(s)**

By "oil" it differs from the pasty compounds or waxes that are described in the present invention, in that the oils are liquid at room temperature (25°C) and atmospheric pressure (1.013.105 Pa or 760 mmHg).

Accordingly, the composition of the present invention comprise greater than or equal to 5% by weight of at least one oil chosen from fatty acid triglycerides, relative to the total weight of the composition.

Preferably, said fatty acid each independently has from 4 to 30 carbon atoms and said fatty acid is linear or branched, saturated or unsaturated.

Triglycerides have the general formula (I):

$$CH_2(OOCR_1)CH(OOCR_2)CH_2(OOCR_3) \quad (I)$$

wherein $R_1$, $R_2$ and $R_3$ are usually of different chain lengths but could be the same, e.g., $C_4$-$C_{30}$ and more usually $C_6$-$C_{24}$.

Examples of triglycerides are given in the CTFA Cosmetic Ingredient Handbook.

Preferred triglycerides are obtained from carboxylic acids of carbon chain length ranging from $C_6$ to $C_{24}$, preferably from $C_6$ to $C_{20}$, and more preferably from $C_6$ to $C_{18}$, linear or branched, saturated or unsaturated, and glycerol.

More preferably, the triglycerides oils of the present invention are chosen from triglycerides of fatty acids containing from 6 to 14 carbon atoms, more preferably from 6 to 12 carbon atoms, in particular from 6 to 10 carbon atoms such as triglycerides of heptanoic acid, 2-ethylhexanoic acid, octanoic acids, caprylic acid, capric acid, or mixtures thereof.

In one embodiment, the triglycerides are synthetic.

In another embodiment, the triglycerides are of plant origin. For example, the plant oils that comprise triglycerides, or triglycerides obtained from the plant oils.

Such vegetable derived triglycerides are particularly preferred, and specific examples of preferred materials as sources of triglycerides include peanut oil, sesame oil, avocado oil, coconut oil, cocoa butter oil, almond oil, safflower oil, corn oil, cotton seed oil, castor oil, olive oil, jojoba oil, palm oil, soybean oil, wheat germ oil, linseed oil, and sunflower seed oil.

Mentions maybe made of the canola oil such as that sold under the tradename Lipex Preact by the company AARHUSKARL SHAMN, or ricinus communis (castor) seed oil sold under the tradename Lipovol CO by the company Vantage Specialty Chemicals.

Preferably, mentions can be made of Caprylic/capric acid triglycerides, such as those sold by the company

Stearineries Dubois or those sold under the names Miglyol® 810, 812 and 818, jojoba oil, and shea butter oil. Mentions may also be made of the product sold by the company Wilmar under the name Wilfare Ster MCT, with INCI name caprylic/capric triglyceride.

Advantageously, the at least one oil chosen from fatty acid triglycerides is present in the composition of the present invention in an amount ranging from 5% to 40% by weight, more preferably from 8% to 20% by weight, relative to the total weight of the composition.

Even more preferably this amount is ranging from 10% to 15% by weight, relative to the total weight of the composition.

According to a preferred embodiment, the composition of the present invention may further comprise additional oils.

Oils may comprise nonvolatile oils and volatile oils. The term "nonvolatile oil" means an oil that remains on the skin or the keratin fibre at room temperature and atmospheric pressure. More specifically, a nonvolatile oil has an evaporation rate strictly less than 0.01 $mg/cm^2/min$.

To measure this evaporation rate, 15 g of oil or of oil mixture to be tested are placed in a crystallizing dish 7 cm in diameter, which is placed on a balance in a large chamber of about 0.3 $m^3$ that is temperature-regulated, at a temperature of 25°C, and hygrometry-regulated, at a relative humidity of 50%. The liquid is allowed to evaporate freely, without stirring it, while providing ventilation by means of a fan (Papst-Motoren, reference 8550 N, rotating at 2700 rpm) placed in a vertical position above the crystallizing dish containing said oil or said mixture, the blades being directed towards the crystallizing dish, 20 cm away from the bottom of the crystallizing dish. The mass of oil remaining in the crystallizing dish is measured at regular intervals. The evaporation rates are expressed in mg of oil evaporated per unit of area ($cm^2$) and per unit of time (minutes).

The term "volatile oil" means any non-aqueous medium that is capable of evaporating on contact with the skin or the lips in less than one hour, at room temperature and atmospheric pressure. The volatile oil is a cosmetic volatile oil, which is liquid at room temperature. More specifically, a volatile oil has an evaporation rate of between 0.01 and 200 $mg/cm^2/min$, limits included.

The oils may be silicone oil, fluoro oil, hydrocarbon-based oil, or a mixture thereof.

For the purposes of the present invention, the term "silicone oil" means an oil comprising at least one silicon atom, and especially at least one Si-O group.

The term "fluoro oil" means an oil comprising at least one fluorine atom.

The term "hydrocarbon-based oil" means an oil mainly containing hydrogen and carbon atoms.

The oils may optionally comprise oxygen, nitrogen, sulfur and/or phosphorus atoms, for example in the form of hydroxyl or acid radicals.

The oil may be chosen from polar oils, apolar oils, or mixtures thereof.

For the purposes of the present invention, the term "polar oil" means an oil whose solubility parameter at 25°C, $\delta a$, is other than 0 $(J/cm^3)^{1/2}$.

For the purposes of the present invention, the term "apolar oil" means an oil whose solubility parameter at 25°C, $\delta a$, is equal to 0 $(J/cm^3)^{1/2}$.

The definition and calculation of the solubility parameters in the Hansen three-dimensional solubility space are described in the article by C.M. Hansen: "The three dimensional solubility parameters", J. Paint Technol. 39, 105 (1967).

According to this Hansen space:

- $\delta D$ characterizes the London dispersion forces derived from the formation of dipoles induced during molecular impacts;
- $\delta p$ characterizes the Debye interaction forces between permanent dipoles and also the Keesom interaction forces between induced dipoles and permanent dipoles;
- $\delta h$ characterizes the specific interaction forces (such as hydrogen bonding, acid/base, donor/acceptor, etc.); and
- $\delta a$ is determined by the equation: $\delta a = (\delta p^2 + \delta h^2)^{½}$.

The parameters $\delta p$, $\delta h$, $\delta D$ and $\delta a$ are expressed in $(J/cm^3)^{½}$.

According to a more preferred embodiment, the additional oils, when existing, are chosen from nonvolatile hydrocarbon-based oil, more preferably of plant origin.

In particular, the nonvolatile hydrocarbon-based oil of plant origin may be chosen from the list of oils below, and mixtures thereof:

- plant oils which are different from the ones described before, such as linum usitatissimum (linseed) oil, macadamia ternifolia seed oil, limnanthes alba (meadowfoam) seed oil, oryza sativa (rice) bran oil, bertholletia excels seed oil.
- nonvolatile oils of high molecular mass, which are of plant origin or obtained from plants, for example between 350 and 10 000 g/mol, for instance:

a) linear fatty acid esters with a total carbon number ranging from 35 to 70, for instance pentaerythrityl tetrapelargonate (MW = 697.05 g/mol),

b) hydroxylated esters such as polyglycerol-2 triisostearate (MW = 965.58 g/mol);

c) esters of $C_{24}$-$C_{28}$ branched fatty acids or fatty alcohols such as those described in patent application EP-A-0 955 039, and especially triisoarachidyl citrate (MW = 1033.76 g/mol), pentaerythrityl tetraisononanoate (MW = 697.05 g/mol), glyceryl triisostearate (MW = 891.51 g/mol), glyceryl tris(2-decyl)tetradecanoate (MW = 1143.98 g/mol), pentaerythrityl tetraisostearate (MW = 1202.02 g/mol), polyglyceryl-2 tetraisostearate (MW = 1232.04 g/mol) or else pentaerythrityl tetrakis(2-decyl)tetradecanoate (MW = 1538.66 g/mol),

- and mixtures thereof.

According to a preferred embodiment, the amount of the oils, including the fatty acid triglycerides as described above, in the composition of the present invention may range from 10% to 90% by weight, preferably from 30% to 85% by weight, relative to the total weight of the composition.

## Pasty compound(s)

The term "pasty compound" used herein is understood to mean a lipophilic fatty compound with a reversible solid/liquid change of state exhibiting, in the solid state, an anisotropic crystalline arrangement and comprising, at a temperature of 23°C, a liquid fraction and a solid fraction.

In other words, the starting melting temperature of the pasty compound is less than 23°C. The liquid fraction of the pasty compound, measured at 23°C, represents 9 to 97% by weight of the composition. This liquid fraction at 23°C preferably represents between 15 and 85%, more preferably between 40 and 85%, by weight.

The melting point of a solid fatty substance can be measured using a differential scanning calorimeter (DSC), for example the calorimeter sold under the name "DSC Q100" by the company TA Instruments with the software "TA Universal Analysis".

The measurement protocol is as follows:

A solid fat sample of about 5 mg is placed in a crucible "hermetic aluminum capsule" When the solid fatty substance is soft (pasty fatty compound), the sample is subjected to a first rise in temperature ranging from 20 °C to 80 °C, at the heating rate from 2 °C / minute to 80 °C, then left to the isotherm of 80 °C for 20 minutes, then cooled from 80 °C to -80 °C at a cooling rate of 2 °C / minute, and finally subjected to a second temperature rise from -80 °C to 20 °C at a heating rate of 2 °C / minute.

The melting temperature value of the solid fatty substance is the value of the peak of the most endothermic peak of the observed melting curve, representing the variation of the difference in power absorbed as a function of temperature."

The ester of dimer dilinoleic acid and polyol(s) or an ester thereof according to the present invention has the general formula (I) below:

$$R_3\text{-OCO-}R_1(\text{-COO-}R_2\text{-OCO-}R_1)_n\text{-COO-}R_3 \qquad (I)$$

in which:

- $COR_1CO$ represents a dimer dilinoleate residue,
- $OR_2O$ represents a fatty alcohol dimer residue ranging from $C_{16}$ to $C_{68}$, especially from $C_{24}$ to $C_{44}$, in particular from $C_{32}$ to $C_{40}$ and more particularly may be of $C_{36}$.
- $OR_3$ represents a monoalcohol residue ranging from 4 to 40 carbon atoms, in particular from 6 to 36 carbon atoms, in particular from 8 to 32, in particular from 16 to 28, and more particularly from 18 to 24 carbon atoms, and
- n is an integer ranging from 1 to 15, in particular from 2 to 10 and more particularly from 5 to 7.

According to one embodiment, $OR_2O$ may represent a dimer dilinoleyl residue.

Moreover, $OR_3$ may represent a hydrocarbon-based monoalcohol residue chosen, for example, from behenyl, isostearyl and phytosteryl residues, and mixtures thereof.

According to another embodiment, the ester of dimer dilinoleic acid and polyol(s) or an ester thereof that may be suitable for use in the invention may, especially, have the general formula (II) below:

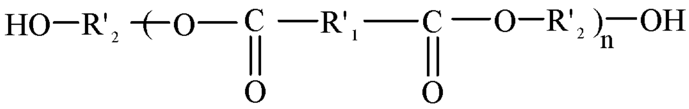

(II)

in which:

- n is an integer ranging from 1 to 15, especially from 2 to 10 and in particular from 5 to 7;
- $OCR'_1CO$ represents a dimer dilinoleate residue,
- $OR'_2O$ represents a diglyceryl residue of general formula (III) below:

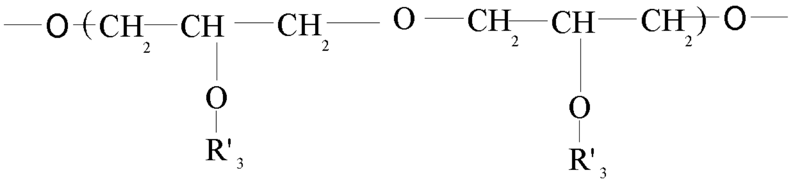

(III)

in which:
$R'_3$ represents H or $OR'_3$ represents a fatty acid residue that may range from $C_8$ to $C_{34}$, especially from $C_{12}$ to $C_{22}$, in particular of $C_{16}$ to $C_{20}$ and more particularly may be $C_{18}$.

According to one embodiment, the fatty acid residue represented by $OR_3$ may be an isostearyl residue.

The viscosity of an ester of dimer dilinoleic acid and polyol(s) or an ester thereof, according to the invention, may be measured according to any process known to those skilled in the art, and especially according to the conventional process described hereinbelow.

The viscosity may be measured using a cone/plate or parallel plate viscometer of Ares type (TA-Instrument) operating in kinetic sweep mode over a shear range of about 1-1000 $s^{-1}$ to induce a flow tension at about 1000 Pa.

The cone/plate or parallel plates may consist of a material selected from the group constituted of stainless steel, acrylic resins or polyphenylene sulfide (PPS resin).

The cone/plate diameter may be 25 mm (cone angle 0.10 radiants).

The measurement is performed at about 25°C.

Before any measurement, the stability of the sample is checked by means of the dynamic sweep period test, which makes it possible to determine if the sample is stable per se.

The shear viscosity is determined using the ETA value in the plateau region according to the flow.

The dynamic sweep period is determined at a frequency of 1.0 Hz over a period of 600 seconds.

The measurements at constant sweep rate are performed with a rate ranging from 1.0 to 1000 $s^{-1}$ and in particular from 1.0 to 100 $s^{-1}$.

The viscosity of an ester of dimer dilinoleic acid and polyol(s) or an ester thereof suitable for use in the invention may range from about 20 000 mPa.s to about 150 000 mPa.s, especially from about 40 000 mPa.s to about 100 000 mPa.s and in particular from about 60 000 mPa.s to about 80 000 mPa.s.

An ester of dimer dilinoleic acid and polyol(s) or an ester thereof that is suitable for the invention may be chosen especially from the esters having the following INCI nomenclature: polyglyceryl-2 isostearate dimer dilinoleate copolymer, bis-behenyl/isostearyl/phytosteryl dimer dilinoleyl dimer dilinoleate, and mixtures thereof.

Such compounds may be obtained, for example, under the reference Hailucent ISDA (Kokyu Alcohol) and Plandool-G (Nippon Fine Chemical Company Ltd).

An ester of dimer dilinoleic acid and polyol(s) or an ester thereof suitable for use in the invention may be advantageously present in the composition according to the invention in an amount sufficient to give these compositions improved cosmetic properties, especially in terms of mean gloss staying power.

Advantageously, the at least one pasty compound chosen from an ester of dimer dilinoleic acid and polyol(s) or an ester thereof is present in the composition of the present invention in an amount ranging from 1% to 40% by weight, relative to the total weight of the composition.

According to an embodiment, the at least one pasty compound chosen from an ester of dimer dilinoleic acid and polyol(s) or an ester thereof is present in the composition of the present invention in an amount ranging from 1% to 30% by weight, more preferably from 5% to 20% by weight, relative to the total weight of the composition.

Alternatively, the composition according to the present invention can further comprises additional pasty compound chosen from polyol ethers, which is, in particular, polyalkylene glycol pentaerythritol ethers.

The polyalkylene glycol pentaerythritol ethers may contain in particular from 1 to 450 oxyalkylenated units,

preferably from 1 to 200 oxyalkylenated units, even better from 1 to 100 oxyalkylenated units and better still from 1 to 50 oxyalkylenated units. They may be chosen in particular from polyethylene glycol pentaerythritol ethers containing from 1 to 450 oxyethylenated units, preferably from 1 to 200 oxyethylenated units, even better from 1 to 100 oxyethylenated units and better still from 1 to 50 oxyethylenated units; polypropylene glycol pentaerythritol ethers containing from 1 to 450 oxypropylenated units, preferably from 1 to 200 oxypropylenated units, even better from 1 to 100 oxypropylenated units and better still from 1 to 50 oxypropylenated units; and mixtures thereof. According to a preferred embodiment of the invention, use is made of the polyethylene glycol pentaerythrityl ether containing 5 oxyethylenated units (5 EO) (CTFA name : PEG-5 Pentaerythrityl Ether), the polypropylene glycol pentaerythritol ether containing 5 oxypropylenated units (5 PO) (CTFA name: PPG-5 Pentaerythrityl Ether), and mixtures thereof, and more especially the PEG-5 Pentaerythrityl Ether, PPG-5 Pentaerythrityl Ether and soya bean oil mixture marketed under the name "Lanolide" by the company Vevy, a mixture in which their constituents are in a 46/46/8 weight ratio: 46% of PEG-5 Pentaerythrityl Ether, 46% of PPG-5 Pentaerythrityl Ether and 85% of soya bean oil.

More preferably the composition of the present invention comprises at least one pasty compound chosen from polyol ethers comprising polyethylene glycol pentaerythrityl ether containing 5 oxyethylenated units, polypropylene glycol pentaerythritol ether containing 5 oxypropylenated units, and mixtures thereof.

Even more preferably, the pasty compound is a polyol ether in the form of a mixture comprising polyethylene glycol pentaerythritol ether comprising 5 oxyethylenated units, polypropylene glycol pentaerythritol ether comprising 5 oxypropylenated units, and soya bean oil.

Such products are commercially available, for example, marketed under the name "Lanolide" by the company Vevy, a mixture in which their constituents are in a 46/46/8 weight ratio: 46% of PEG-5 Pentaerythrityl Ether, 46% of PPG-5 Pentaerythrityl Ether and 8% of soya bean oil.

The composition of the present invention can further comprise at least one chosen from hydrocarbon based pasty compounds and more particularly from the following compounds:

- lanolin and derivatives thereof;
- petroleum jelly, in particular the product whose INCI name is petrolatum and which is sold under the name Ultima White PET USP by the company Calumet Specialty,
- vinyl polymers, especially:
  - olefin homopolymers and copolymers,
  - hydrogenated diene homopolymers and copolymers,
  - linear or branched oligomers, homopolymers and copolymers of alkyl (meth)acrylates preferably containing a $C_8$-$C_{30}$ alkyl group,
  - oligomers, homopolymers and copolymers of vinyl esters containing $C_8$-$C_{30}$ alkyl groups, such as vinyl ester homopolymers containing $C_8$-$C_{30}$ alkyl groups, such as polyvinyl laurate (sold especially under the reference Mexomer PP by the company Chimex) and arachidyl propionate sold under the brand name Waxenol 801 by Alzo;
  - oligomers, homopolymers and copolymers of vinyl ethers containing $C_8$-$C_{30}$ alkyl groups;
- liposoluble polyethers resulting from the polyetherification between one or more $C_2$-$C_{100}$ and preferably $C_2$-$C_{50}$ diols, among the liposoluble polyethers that are particularly preferred are copolymers of ethylene oxide and/or of propylene oxide with $C_6$-$C_{30}$ long-chain alkylene oxides, more preferably such that the weight ratio of the ethylene oxide and/or of the propylene oxide to the alkylene oxides in the copolymer is from 5:95 to 70:30. In this family, mention will be made especially of copolymers such that the long-chain alkylene oxides are arranged in blocks having an average molecular weight from 1000 to 10 000, for example a polyoxyethylene/polydodecyl glycol block copolymer such as the ethers of dodecanediol (22 mol) and of polyethylene glycol (45 OE) sold under the brand name Elfacos ST9 by Akzo Nobel.
  - esters especially those chosen from:
    - esters of a glycerol oligomer, especially diglycerol esters, in particular condensates of adipic acid and of glycerol, for which some of the hydroxyl groups of the glycerols have reacted with a mixture of fatty acids such as stearic acid, capric acid, stearic acid and isostearic acid, and 12-hydroxystearic acid, preferably such as bis-diglyceryl polyacyladipate-2 sold under the brand name Softisan® 649 by the company Cremer Oleo,
    - vinyl ester homopolymers containing $C_8$-$C_{30}$ alkyl groups, such as polyvinyl laurate (sold especially under the reference Mexomer PP by the company Chimex) and arachidyl propionate sold under the brand name Waxenol 801 by Alzo,
    - phytosterol esters,

- fatty acid triglycerides and derivatives thereof, for instance triglycerides of fatty acids, which are especially $C_{16}$-$C_{24}$, and partially or totally hydrogenated such as those sold under the reference Softisan 100 by the company Sasol,
- pentaerythritol esters,
- noncrosslinked polyesters resulting from polycondensation between a linear or branched $C_4$-$C_{50}$ dicarboxylic acid or polycarboxylic acid and a $C_2$-$C_{50}$ diol or polyol,
- aliphatic esters of an ester, resulting from the esterification of an aliphatic hydroxycarboxylic acid ester with an aliphatic carboxylic acid comprising from 4 to 30 carbons, preferably 8 to 30 carbons. It is preferably chosen from hexanoic acid, heptanoic acid, octanoic acid, 2-ethylhexanoic acid, nonanoic acid, decanoic acid, undecanoic acid, dodecanoic acid, tridecanoic acid, tetradecanoic acid, pentadecanoic acid, hexadecanoic acid, hexyldecanoic acid, heptadecanoic acid, octadecanoic acid, isostearic acid, nonadecanoic acid, eicosanoic acid, isoarachidic acid, octyldodecanoic acid, heneicosanoic acid and docosanoic acid, and mixtures thereof. The aliphatic carboxylic acid is preferably branched. The aliphatic hydroxycarboxylic acid ester is advantageously derived from a hydroxylated aliphatic carboxylic acid containing from 2 to 40 carbon atoms, preferably from 10 to 34 carbon atoms and better still from 12 to 28 carbon atoms, and from 1 to 20 hydroxyl groups, preferably from 1 to 10 hydroxyl groups and better still from 1 to 6 hydroxyl groups. The aliphatic hydroxycarboxylic acid ester is chosen from:

    o partial or total esters of saturated linear mono-hydroxylated aliphatic monocarboxylic acids;
    o partial or total esters of unsaturated monohydroxylated aliphatic monocarboxylic acids;
    o partial or total esters of saturated monohydroxylated aliphatic polycarboxylic acids;
    o partial or total esters of saturated polyhydroxylated aliphatic polycarboxylic acids;
    o partial or total esters of $C_2$ to $C_{16}$ aliphatic polyols that have reacted with a monohydroxylated or polyhydroxylated aliphatic monocarboxylic or polycarboxylic acid,
    o and mixtures thereof.

- mango butter, such as the product sold under the reference Lipex 203 by the company AarhusKarlshamn,
- shea butter in particular the product whose INCI name is Butyrospermum parkii Butter, such as the product sold under the reference Sheasoft® by the company Aarhuskarlshamn,
- and mixtures thereof.

According to a preferred embodiment, the amount of the pasty compound, including the ester of dimer dilinoleic acid and polyol(s) or an ester thereof as described above, in the composition of the present invention may range from 1% to 50% by weight, more preferably from 5% to 40% by weight, relative to the total weight of the composition.

**Wax(es)**

The wax under consideration in the context of the present invention is generally a lipophilic compound that is solid at room temperature (25°C), with a solid/liquid reversible than or equal to 40°C, which may be up to 200°C and in particular up to 120°C.
The melting point of can be measured using a differential scanning calorimeter (DSC), for example the calorimeter sold under the name "DSC Q100" by the company TA Instruments with the software " TA Universal Analysis " according to the protocol already described.
The composition of the present invention comprises at least one wax of solid linear ester derived from $C_6$- $C_{30}$ fatty acid, chosen from the group comprising stearyl stearate, tetradecyl tetradecanoate (INCI name: myristyl myristate), cetyl myristate, stearyl myristate, myristyl palmitate, stearyl palmitate, myristyl stearate, cetyl stearate, stearyl stearate and cetyl palmitate, and mixtures thereof.
As an example of waxes of solid linear ester that are most particularly suitable for the invention, mention may be made of cetyl palmitate, which is for example sold under the name OLEM CP by the company Suzhou Eleco Chemical Industry.
According to one preferred embodiment, the composition according to the invention comprises the wax(es) of solid linear ester(s) in an amount ranging from 0.5% to 20% by weight, preferably from 1% to 10% by weight, relative to the total weight of the composition.
According to a preferred embodiment, the composition of the present invention may further comprise an additional wax, which is different from the solid linear esters as described above.
The waxes that may be used in a composition according to the present invention are chosen from solid waxes that may or may not be deformable at room temperature of animal, plant, mineral or synthetic origin, and mixtures thereof, preferably chosen from wax of animal or plant origin.

Hydrocarbon-based waxes, for instance beeswax, lanolin wax or Chinese insect wax; rice wax, carnauba wax, candelilla wax, ouricury wax, esparto grass wax, cork fibre wax, sugar cane wax, Japan wax and sumach wax, helianthus annuus (sunflower) seed wax; montan wax, microcrystalline waxes, paraffins and ozokerite; polyethylene waxes, polymethylene waxes, the waxes obtained by Fisher-Tropsch synthesis and waxy copolymers, and also esters thereof, may especially be used.

Mentions may be especially made of beeswax, for example the product sold under the trade name Cire d'abelle blanche BR G889 by Koster Keunen. carnauba wax, for example sold under the name Cerauba T1 Bio by the company Baerlocher. helianthus annuus (sunflower) seed wax sold under the name Sunflower Wax by the company Koster Keunen, or a mixture thereof.

Mention may also be made of waxes obtained by catalytic hydrogenation of animal or plant oils containing linear or branched $C_8$-$C_{32}$ fatty chains.

Among these waxes that may especially be mentioned are hydrogenated jojoba oil, hydrogenated sunflower oil, hydrogenated castor oil, hydrogenated coconut oil, hydrogenated lanolin oil, bis(1,1,1-trimethylolpropane) tetrastearate sold under the name Hest 2T-4S by the company Heterene, and bis(1,1,1-trimethylolpropane) tetrabehenate sold under the name Hest 2T-4B by the company Heterene.

As for esters obtained by catalytic hydrogenation of animal or plant oils, mention may be made to the waxes obtained by hydrogenation of palm oil esterified with cetyl alcohol. Such products can be found, for example, under the trade name CUTINA® CP from Cognis (BASF), castor oil esterified with cetyl alcohol, sold under the names Phytowax ricin 16L64® and 22L73® by the company Sophim, may also be used. Such waxes are described in patent application FR-A-2 792 190.

A wax that may be used is a $C_{20}$-$C_{40}$ alkyl (hydroxystearyloxy)stearate (the alkyl group containing from 20 to 40 carbon atoms), alone or as a mixture. Such a wax is especially sold under the names Kester Wax K 82 P®, Hydroxypolyester K 82 P® and Kester Wax K 80 P® by the company Koster Keunen.

Advantageously, the wax(es) suitable for the present invention is chosen from hydrocarbon-based waxes, esters obtained by catalytic hydrogenation of animal or plant oils, or a mixture thereof.

More preferably, the composition of the present invention optionally comprises at least one additional wax chosen from beeswax, carnauba wax, helianthus annuus (sunflower) seed wax, or a mixture thereof.

If the composition comprises the additional wax(es), the amount of all the wax(es) including the solid linear ester as described above ranges from 1% and 25%, preferably from 5% to 20% by weight, relative to the total weight of the composition.

According to a preferred embodiment, the weight ratio of the at least one oil chosen from fatty acid triglycerides: the at least one pasty compound(s) chosen from an ester of dimer dilinoleic acid and polyol(s) or an ester thereof ranges from 1:3 to 3:1, preferably from 1:2 to 2:1, more preferably from 4:5 to 6:5.

## Dyestuff

The solid anhydrous composition in accordance with the present invention may optionally comprise at least one dyestuff, which may be chosen from water-soluble or water-insoluble, liposoluble or non-liposoluble, organic or mineral dyestuffs, and materials with an optical effect, and mixtures thereof. Preferably, the amount of dyestuff is below 10 % by weight relative to the total weight of the composition.

For the purposes of the present invention, the term "dyestuff" means a compound that is capable of producing a coloured optical effect when it is formulated in sufficient amount in a suitable cosmetic medium.

Preferably, the composition according to the present invention comprises at least one dyestuff, chosen from pigments and/or nacres and/or water-soluble dyes, and mixtures thereof.

According to one preferred embodiment, a composition according to the present invention comprises at least one water-soluble dyestuff.

The water-soluble dyestuffs used according to the present invention are more particularly water-soluble dyes.

For the purposes of the present invention, the term "water-soluble dye" is intended to mean any natural or synthetic, generally organic compound, which is soluble in an aqueous phase or water-miscible solvents and which is capable of colouring. In particular, the term "water-soluble" is intended to mean the capacity of a compound to be dissolved in water, measured at 25°C, to a concentration at least equal to 0.1 g/l (production of a macroscopically isotropic, transparent, coloured or colourless solution). This solubility is in particular greater than or equal to 1 g/l.

As water-soluble dyes that are suitable for use in the present invention, mention may be made in particular of synthetic or natural water-soluble dyes, for instance FDC Red 4 (CI: 14700), DC Red 6 (Lithol Rubine Na; CI: 15850), DC Red 22 (CI: 45380), DC Red 28 (CI: 45410 Na salt), DC Red 30 (CI: 73360), DC Red 33 (CI: 17200), DC Orange 4 (CI: 15510), FDC Yellow 5 (CI: 19140), FDC Yellow 6 (CI: 15985), DC Yellow 8 (CI: 45350 Na salt), FDC Green 3 (CI: 42053), DC Green 5 (CI: 61570), FDC Blue 1 (CI: 42090).

As non-limiting illustrations of sources of water-soluble dyestuff(s) that may be used in the context of the present

invention, mention may be made especially of those of natural origin, such as extracts of cochineal carmine, of beetroot, of grape, of carrot, of tomato, of annatto, of paprika, of henna, of caramel and of curcumin.

Thus, the water-soluble dyestuffs that are suitable for use in the present invention are especially carminic acid, betanin, anthocyans, enocyanins, lycopene, β-carotene, bixin, norbixin, capsanthin, capsorubin, flavoxanthin, lutein, cryptoxanthin, rubixanthin, violaxanthin, riboflavin, rhodoxanthin, cantaxanthin and chlorophyll, and mixtures thereof.

They may also be copper sulfate, iron sulfate, water-soluble sulfopolyesters, rhodamine, betaine, methylene blue, the disodium salt of tartrazine and the disodium salt of fuchsin.

Some of these water-soluble dyestuffs are in particular permitted for food use. Representatives of these dyes that may be mentioned more particularly include dyes of the carotenoid family, referenced under the food codes E120, E162, E163, E160a-g, E150a, E101, E100, E140 and E141.

According to one preferred variant, the water-soluble dyestuff(s) that are to be transferred onto the skin and/or the lips intended to be made up are formulated in a physiologically acceptable medium so as to be compatible with impregnation into the substrate.

The water-soluble dyestuff(s) may be present in the composition according to the present invention in a content ranging from 0.001% to 5% by weight and preferably from 0.002% to 3% by weight relative to the total weight of the composition.

According to another embodiment, the composition according to the present invention may comprise at least one pigment and/or nacre as dyestuff.

The term "pigments" should be understood as meaning white or coloured, inorganic (mineral) or organic particles, which are insoluble in the liquid organic phase, and which are intended to colour and/or opacify the composition and/or the deposit produced with the composition.

The pigments may be chosen from mineral pigments, organic pigments and composite pigments (i.e. pigments based on mineral and/or organic materials).

The pigments may be chosen from monochromatic pigments, lakes, nacres, and pigments with an optical effect, for instance reflective pigments and goniochromatic pigments.

The mineral pigments may be chosen from metal oxide pigments, chromium oxides, iron oxides, titanium dioxide, zinc oxides, cerium oxides, zirconium oxides, manganese violet, Prussian blue, ultramarine blue and ferric blue, and mixtures thereof.

The organic pigments may be, for example:

- cochineal carmine,
- organic pigments of azo dyes, anthraquinone dyes, indigoid dyes, xanthene dyes, pyrene dyes, quinoline dyes, triphenylmethane dyes or fluoran dyes,
- organic lakes or insoluble sodium, potassium, calcium, barium, aluminum, zirconium, strontium or titanium salts of acidic dyes such as azo, anthraquinone, indigoid, xanthene, pyrene, quinoline, triphenylmethane or fluoran dyes. These dyes generally comprise at least one carboxylic or sulfonic acid group;
- melanin-based pigments.

Among the organic pigments, mention may be made of D&C Blue No. 4, D&C Brown No. 1, D&C Green No. 5, D&C Green No. 6, D&C Orange No. 4, D&C Orange No. 5, D&C Orange No. 10, D&C Orange No. 11, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 33, D&C Red No. 34, D&C Red No. 36, D&C Violet No. 2, D&C Yellow No. 7, D&C Yellow No. 8, D&C Yellow No. 10, D&C Yellow No. 11, FD&C Blue No. 1, FD&C Green No. 3, FD&C Red No. 40, FD&C Yellow No. 5 and FD&C Yellow No. 6.

For the purposes of the present patent application, the term "nacre" is intended to mean coloured particles of any form, which may or may not be iridescent, in particular produced by certain molluscs in their shell, or alternatively synthesized, and which have a colour effect via optical interference.

Examples of nacres that may be mentioned include nacreous pigments such as titanium mica coated with an iron oxide, mica coated with bismuth oxychloride, titanium mica coated with chromium oxide, titanium mica coated with an organic dye in particular of the abovementioned type, and also nacreous pigments based on bismuth oxychloride. They may also be mica particles, at the surface of which are superposed at least two successive layers of metal oxides and/or of organic dyestuffs.

The nacres may more particularly have a yellow, pink, red, bronze, orangey, brown, gold and/or coppery colour or tint.

As illustrations of nacres that may be introduced as interference pigments into the composition, mention may be made of the gold-coloured nacres sold in particular by the company Engelhard under the name Brilliant gold 212G (Timica), Gold 222C (Cloisonne), Sparkle gold (Timica), Gold 4504 (Chromalite) and Monarch gold 233X (Cloisonne); the bronze nacres sold in particular by the company Merck under the name Bronze fine (17384) (Colorona) and Bronze

(17353) (Colorona) and by the company Engelhard under the name Super bronze (Cloisonne); the orange nacres sold in particular by the company Engelhard under the name Orange 363C (Cloisonne) and Orange MCR 101 (Cosmica) and by the company Merck under the name Passion orange (Colorona) and Matte orange (17449) (Microna); the brown nacres sold in particular by the company Engelhard under the name Nu-antique copper 340XB (Cloisonne) and Brown CL4509 (Chromalite); the nacres with a copper tint sold in particular by the company Engelhard under the name Copper 340A (Timica); the nacres with a red tint sold in particular by the company Merck under the name Sienna fine (17386) (Colorona); the nacres with a yellow tint sold in particular by the company Engelhard under the name Yellow (4502) (Chromalite); the red nacres with a gold tint sold in particular by the company Engelhard under the name Sunstone G012 (Gemtone); the pink nacres sold in particular by the company Engelhard under the name Tan opale G005 (Gemtone); the black nacres with a gold tint sold in particular by the company Engelhard under the name Nu antique bronze 240 AB (Timica), the blue nacres sold in particular by the company Merck under the name Matte blue (17433) (Microna), the white nacres with a silvery tint sold in particular by the company Merck under the name Xirona Silver, and the golden-green pink-orange nacres sold in particular by the company Merck under the name Indian summer (Xirona), and mixtures thereof.

## Additives

In a particular embodiment, a solid anhydrous composition according to the present invention may further comprise at least one additive usually used in the field under consideration. In particular the additive is chosen from gums, anionic, cationic, amphoteric or nonionic surfactants, silicone surfactants, resins, thickening agents, dispersants, antioxidants, preserving agents, fragrances, neutralizers, antiseptics, additional cosmetic active agents, such as vitamins, moisturizers, emollients or collagen-protecting agents, and mixtures thereof.
It is a matter of routine operations for a person skilled in the art to adjust the nature and amount of the additives present in the compositions in accordance with the present invention such that the advantageous properties of the composition used according to the present invention are not, or are not substantially, adversely affected by the envisaged addition.
According to a preferred embodiment, the invention relates to a solid anhydrous composition comprising:

a) greater than or equal to 5% by weight of caprylic/capric acid triglycerides, relative to the total weight of the composition,
b) Bis-Behenyl/Isostearyl/Phytosteryl Dimer Dilinoleyl Dimer Dilinoleate, and
c) cetyl palmitate.

## Galenic form

The composition of the present invention is suitable to be used as a skin care, make up or cosmetic treatment product. More particularly, the composition of the present invention is in the form of make up product such as lipstick and so on.
The composition according to the present invention may be prepared in a conventional manner.
The terms "between" and "ranging from" used herein should be understood as including the limits.
The present invention also relates to a process for caring for/making up keratin materials such as the skin and the lips, preferably the lips, by applying the solid anhydrous composition as described above to the keratin materials.
Unless defined otherwise, all technical and scientific terms used herein have the same meaning as commonly understand by one of ordinary skill in the art to which the present invention pertains.
The examples that follow are given as non-limiting illustrations of the present invention. The percentages are weight percentages.

## Examples

Formulation examples

The following compositions are prepared (the contents are expressed as weight percentages of active material, unless otherwise indicated):

| INCI name | Content | | |
|---|---|---|---|
| | Invention Example 1 | Invention Example 2 | Comparative Example 1 (without pasty compound) |
| CAPRYLIC/CAPRIC TRIGLYCERIDE (Wilfare Ster MCT from Wilmar) | 12.5 | 7 | 7 |
| BIS-BEHENYL/ISOSTEARYL/PHYTOSTERYL DIMER DILINOLEYL DIMER DILINOLEATE (Plandool G from Nippon Fine Chemical Co., Ltd.) | 14.5 | 6 | 0 |
| CETYL PALMITATE (OLEM CP from Suzhou Eleco Chemical Industry) | 4.5 | 3 | 3 |
| CANOLA OIL (Lipex Preact from AARHUSKARL SHAMN) | 33.7 | 49.2 | 55.2 |
| RICINUS COMMUNIS (CASTOR) SEED OIL (Lipovol CO from Vantage Specialty Chemicals) | 12.5 | 12.5 | 12.5 |
| POLYGLYCERYL-2 TRIISOSTEARATE | 10 | 10 | 10 |
| HELIANTHUS ANNUUS (SUNFLOWER) SEED WAX (Sunflower Wax from Koster Keunen) | 6.3 | 6.3 | 6.3 |
| COPERNICIA CERIFERA (CARNAUBA) WAX (Cerauba T1 Bio from Baerlocher) | 1.5 | 1.5 | 1.5 |
| BEESWAX (Cire d'abelle blanche BR G889 from Koster Keunen) | 2 | 2 | 2 |
| RED 28 LAKE | 2.5 | 2.5 | 2.5 |

Protocol of preparation

The above listed compositions are prepared following the steps of:

mixing all the ingredients under 93°C, stiring the mixture at 300r/min by IKA Blender Euro - ST P CV S25 model until homogeneous;
pouring the homogenized mixture into a lip stick mould at 93 °C, leaving the mixture in the mould under 25 °C until solidation;
demoulding the solid mixture from the lip stick mould.

Evaluation example

Evaluation on the hardness, stability of the shape, as well as usage effect such as spreadability, and homogeneous wear, non-sticky feeling after application of the compositions according to invention and comparative examples are performed.

Hardness is evaluated according to the protocol described previously.

Stability of the shape is evaluated using crash test, by following steps:

heat the composition to 38 °C for 24 hours;
apply the composition to the lips under the heated temperature.

Spreadability is evaluated by 5 experts by the following steps:

repeatly apply the composition three times on the same area of the forearm using the same force;
weigh the weight loss of the composition;
measure the size of the area on the forearm where the composition is applied;
calculate the weight loss per square centimeter.

Finally, comments or scores are given by the experts on the above mentioned properties.

5: very good;
4: basically good;
3: acceptable;
2: slightly poor and not acceptable;
1: poor, not acceptable.

The non-sticky feeling and homogeneous wear after application is evaluated by 5 experts by the following steps:

Firstly, repeatly apply the compositions according to invention and comparative examples, respectively, three times on the same area of the lips using the same force;
Then comments or scores are given by the experts on the above mentioned properties.
5: very good;
4: basically good;
3: acceptable;
2: slightly poor and not acceptable;
1: poor, not acceptable.

| Properties | Scores of the examples | | |
|---|---|---|---|
| | Invention Example 1 | Invention Example 2 | Comparative Example 1 |
| Hardness | 5 | 5 | 1 |
| Stability of the shape | Stable over 10 stroke | Stable over 10 stroke | Too soft |
| Spreadability | 5 | 5 | 2 |
| Homogeneous wear | 5 | 5 | 2 |
| Non-sticky feeling | 4 | 5 | 5 |

It is observed that the compositions according to invention examples 1-2 possess good stability of shape and usage effect while the composition according to comparative example 1 is too soft. Based on the above listed evaluation results, the inventors discovered that the composition according to the present invention overcomes some technical issues existing in the prior arts, and provides a solid anhydrous composition with desired hardness and good usage.

## Claims

1. A solid anhydrous composition for caring for and/or making up keratin materials comprising,

a) greater than or equal to 5% of at least one oil chosen from fatty acid triglycerides, relative to the total weight of the composition,
b) at least one pasty compound chosen from an ester of dimer dilinoleic acid and polyol(s) or an ester thereof, chosen from the compound of formula (I):

$$R_3\text{-OCO-}R_1(\text{-COO-}R_2\text{-OCO-}R_1)_n\text{COO-}R_3 \qquad (I)$$

in which:

- $COR_1CO$ represents a dimer dilinoleate residue,
- $OR_2O$ represents a fatty alcohol dimer residue ranging from $C_{16}$ to $C_{68}$, especially from $C_{24}$ to $C_{44}$, in particular from $C_{32}$ to $C_{40}$ and more particularly may be of $C_{36}$,
- $OR_3$ represents a monoalcohol residue ranging from 4 to 40 carbon atoms, in particular from 6 to 36 carbon atoms, in particular from 8 to 32, in particular from 16 to 28, and more particularly from 18 to 24 carbon atoms, and
- n is an integer ranging from 1 to 15, in particular from 2 to 10 and more particularly from 5 to 7,

and

c) at least one wax of solid linear ester derived from $C_6$- $C_{30}$ fatty acid chosen from the group comprising stearyl stearate, myristyl myristate, cetyl myristate, stearyl myristate, myristyl palmitate, stearyl palmitate, myristyl stearate, cetyl stearate, stearyl stearate, cetyl palmitate, and mixtures thereof.

**2.** The composition according to claim 1, **characterized in that** it further comprises an additional oil selected from nonvolatile hydrocarbon-based oil of plant origin, more preferably selected from the group consisting of plant oils, nonvolatile oils of high molecular mass between 350 and 10 000 g/mol, and mixtures thereof; more preferably selected from canola oil, ricinus communis seed oil, polyglycerol-2 triisostearate, and mixture thereof.

**3.** The composition according to claim 1 or 2, **characterized in that** it further comprises an additional wax chosen from hydrocarbon-based waxes of plant or animal origin, esters obtained by catalytic hydrogenation of animal or plant oils, or a mixture thereof;
preferably chosen from beeswax, carnauba wax, helianthus annuus (sunflower) seed wax, or a mixture thereof.

**4.** The composition according to any one of claims 1 to 3, **characterized in that** it comprises at least one dyestuff chosen from water-soluble or water-insoluble, liposoluble or non-liposoluble, organic or mineral dyestuffs, and materials with an optical effect, and mixtures thereof.

**5.** The composition according to any one of claims 1-4, **characterized in that** the fatty acid triglycerides are chosen from triglycerides of fatty acids, which are especially $C_6$-$C_{20}$, preferably $C_6$-$C_{14}$, more preferably $C_6$-$C_{12}$, most preferably $C_6$-$C_{10}$; preferably chosen from heptanoic or octanoic acid triglycerides, caprylic/capric acid triglycerides and mixtures thereof.

**6.** The composition according to anyone of claims 1-5, **characterized in that** the pasty compound is Bis-Behenyl/Isostearyl/Phytosteryl Dimer Dilinoleyl Dimer Dilinoleate.

**7.** The composition according to any one of claims 1-6, **characterized in that** the solid linear ester is cetyl palmitate.

**8.** The composition according to any one of claims 1-7, **characterized in that** the at least one oil chosen from fatty acid triglycerides is present in an amount ranging from 5% to 40% by weight, more preferably from 8% to 20% by weight, relative to the total weight of the composition.

**9.** The composition according to any one of claims 1-8, **characterized in that** the at least one chosen from an ester of dimer dilinoleic acid and polyol(s) or an ester thereof is present in an amount ranging from 1% to 40% by weight, preferably from 1% to 30% by weight, more preferably from 5% to 20% by weight, relative to the total weight of the composition.

**10.** The composition according to any one of claims 1-9, **characterized in that** the at least one wax of solid linear ester derived from $C_6$- $C_{30}$ fatty acid is present in an amount ranging from 0.5% to 20% by weight, preferably from 1% to 10% by weight, relative to the total weight of the composition.

**11.** Composition according to any one of the claims 1-10, wherein the weight ratio of the at least one oil chosen from fatty acid triglycerides: the at least one pasty compound(s) chosen from an ester of dimer dilinoleic acid and polyol(s) or an ester thereof ranges from 1:3 to 3:1, preferably from 1:2 to 2:1, more preferably from 4:5 to 6:5.

**12.** A process for caring for and/or making up keratin materials such as the skin and the lips, preferably the lips, by applying the composition as defined in any of claims 1-11 to the keratin materials.

## Patentansprüche

**1.** Feste wasserfreie Zusammensetzung zur Pflege und/oder zum Schminken von Keratinmaterialien, umfassend:

a) größer oder gleich 5% mindestens eines aus Fettsäuretriglyceriden ausgewählten Öls, bezogen auf das Gesamtgewicht der Zusammensetzung,
b) mindestens eine pastöse Zusammensetzung, die aus einem Ester von Dimerdilinolsäure und Polyol(en) oder

einem Ester davon ausgewählt ist, ausgewählt aus der Verbindung der Formel (I):

$$R_3\text{-OCO-}R_1(\text{-COO-}R_2\text{-OCO-}R_1)_n\text{-COO-}R_3 \qquad (I)$$

in der

- $COR_1CO$ einen Dimerdilinoleatrest repräsentiert,
- $OR_2O$ einen Fettalkoholdimerrest im Bereich von $C_{16}$ bis $C_{68}$, speziell von $C_{24}$ bis $C_{44}$, insbesondere von $C_{32}$ bis $C_{40}$ repräsentiert und insbesondere $C_{36}$ sein kann,
- $OR_3$ einen Monoalkoholrest mit 4 bis 40 Kohlenstoffatomen, insbesondere mit 6 bis 36 Kohlenstoffatomen, insbesondere mit 8 bis 32, insbesondere mit 16 bis 28 und spezieller noch mit 18 bis 24 Kohlenstoffatomen repräsentiert, und
- n eine ganze Zahl im Bereich von 1 bis 15, insbesondere von 2 bis 10 und spezieller noch von 5 bis 7 ist, und

c) mindestens ein von $C_6$-$C_{30}$-Fettsäure abgeleitetes Wachs von festem linearem Ester, das aus der Stearylstearat, Myristylmyristat, Cetylmyristat, Stearylmyristat, Myristylpalmitat, Stearylpalmitat, Myristylstearat, Cetylstearat, Stearylstearat, Cetylpalmitat und Mischungen davon umfassenden Gruppe ausgewählt ist.

**2.** Zusammensetzung nach Anspruch 1, **dadurch gekennzeichnet, dass** sie ferner ein zusätzliches Öl umfasst, das ausgewählt ist aus nichtflüchtigem kohlenwasserstoffbasiertem Öl pflanzlichen Ursprungs, mehr bevorzugt ausgewählt aus der aus Pflanzenölen, nichtflüchtigen Ölen von hoher Molekülmasse zwischen 350 und 10.000 g/mol und Mischungen davon bestehenden Gruppe; mehr bevorzugt ausgewählt aus Canolaöl, Samenöl von Ricinus communis, Polyglycerin-2-triisostearat und Mischungen davon.

**3.** Zusammensetzung nach Anspruch 1 oder 2, **dadurch gekennzeichnet, dass** sie ferner ein zusätzliches Wachs umfasst, das ausgewählt ist aus kohlenwasserstoffbasierten Wachsen pflanzlichen oder tierischen Ursprungs, durch katalytische Hydrierung tierischer oder pflanzlicher Öle gewonnenen Estern, oder einer Mischung davon; vorzugsweise ausgewählt aus Bienenwachs, Carnaubawachs, Samenwachs von Helianthus annuus (Sonnenblume) oder einer Mischung davon.

**4.** Zusammensetzung nach einem der Ansprüche 1 bis 3, **dadurch gekennzeichnet, dass** sie mindestens einen Farbstoff umfasst, der ausgewählt ist aus wasserlöslichen oder wasserunlöslichen, fettlöslichen oder nicht fettlöslichen, organischen oder mineralischen Farbstoffen und Materialien mit einem optischen Effekt und Mischungen davon.

**5.** Zusammensetzung nach einem der Ansprüche 1 bis 4, **dadurch gekennzeichnet, dass** die Fettsäuretriglyceride ausgewählt sind aus Triglyceriden von Fettsäuren, die insbesondere $C_6$-$C_{20}$, vorzugsweise $C_6$-$C_{14}$, mehr bevorzugt $C_6$-$C_{12}$, am meisten bevorzugt $C_6$-$C_{10}$ sind; vorzugsweise ausgewählt aus Heptan-oder Octansäuretriglyceriden, Capryl-/Caprinsäuretriglyceriden und Mischungen davon.

**6.** Zusammensetzung nach einem der Ansprüche 1 bis 5, **dadurch gekennzeichnet, dass** es sich bei der pastösen Verbindung um Bis-Behenyl-/Isostearyl-/Phytosteryl-Dimerdilinoleyldimerdilinoleat handelt.

**7.** Zusammensetzung nach einem der Ansprüche 1 bis 6, **dadurch gekennzeichnet, dass** es sich bei dem festen linearen Ester um Cetylpalmitat handelt.

**8.** Zusammensetzung nach einem der Ansprüche 1 bis 7, **dadurch gekennzeichnet, dass** mindestens ein aus Fettsäuretriglyceriden ausgewähltes Öl in einer Menge im Bereich von 5 Gew.-% bis 40 Gew.-%, mehr bevorzugt von 8 Gew.-% bis 20 Gew.-% vorliegt, bezogen auf das Gesamtgewicht der Zusammensetzung.

**9.** Zusammensetzung nach einem der Ansprüche 1 bis 8, **dadurch gekennzeichnet, dass** der mindestens eine aus einem Ester von Dimerdilinolsäure und Polyol(en) oder einem Ester davon ausgewählte Ester in einer Menge im Bereich von 1 Gew.-% bis 40 Gew.-%, vorzugsweise von 1 Gew.-% bis 30 Gew.-%, mehr bevorzugt von 5 Gew.-% bis 20 Gew.-% vorliegt, bezogen auf das Gesamtgewicht der Zusammensetzung.

**10.** Zusammensetzung nach einem der Ansprüche 1 bis 9, **dadurch gekennzeichnet, dass** das mindestens eine von $C_6$-$C_{30}$-Fettsäure abgeleitete Wachs von festem linearem Ester in einer Menge im Bereich von 0,5 Gew.-% bis 20

Gew.-%, vorzugsweise von 1 Gew.-% bis 10 Gew.-% vorliegt, bezogen auf das Gesamtgewicht der Zusammensetzung.

**11.** Zusammensetzung nach einem der Ansprüche 1 bis 10, wobei das Gewichtsverhältnis des mindestens einen aus Fettsäuretriglyceriden ausgewählten Öls zu der mindestens einen pastösen Verbindung, die aus einem Ester von Dimerdilinolsäure und Polyol(en) oder einem Ester davon ausgewählt ist, im Bereich von 1:3 bis 3:1, vorzugsweise von 1:2 bis 2:1, mehr bevorzugt von 4:5 bis 6:5 liegt.

**12.** Verfahren zur Pflege und/oder zum Schminken von Keratinmaterialien wie zum Beispiel der Haut und der Lippen, vorzugsweise der Lippen, durch Auftragen der Zusammensetzung nach einem der Ansprüche 1 bis 11 auf die Keratinmaterialien.

**Revendications**

**1.** Composition solide anhydre de soin et/ou de maquillage de matières kératiniques comprenant,

a) une quantité supérieure ou égale à 5 % d'au moins une huile choisie parmi des triglycérides d'acides gras, par rapport au poids total de la composition,
b) au moins un composé pâteux choisi parmi un ester de dimère d'acide dilinoléique et polyol(s) ou un ester de ceux-ci, choisi parmi le composé de formule (1) :

$$R_3\text{-OCO-}R_1(\text{-COO-}R_2\text{-OCO-}R_1)_n\text{-COO-}R_3 \qquad (I)$$

dans laquelle :

- $COR_1CO$ représente un résidu de dimère dilinoléate,
- $OR_2O$ représente un résidu de dimère d'alcool gras allant de $C_{16}$ à $C_{68}$, notamment de $C_{24}$ à $C_{44}$, en particulier de $C_{32}$ à $C_{40}$ et plus particulièrement peut être en $C_{36}$,
- $OR_3$ représente un résidu de monoalcool allant de 4 à 40 atomes de carbone, en particulier de 6 à 36 atomes de carbone, en particulier de 8 à 32, en particulier de 16 à 28, et plus particulièrement de 18 à 24 atomes de carbone, et
- n est un nombre entier allant de 1 à 15, en particulier de 2 à 10 et plus particulièrement de 5 à 7, et

c) au moins une cire d'ester linéaire solide dérivé d'acide gras en $C_6$-$C_{30}$ choisi dans le groupe comprenant du stéarate de stéaryle, myristate de myristyle, myristate de cétyle, myristate de stéaryle, palmitate de myristyle, palmitate de stéaryle, stéarate de myristyle, stéarate de cétyle, stéarate de stéaryle, palmitate de cétyle, et de mélanges de ceux-ci.

**2.** Composition selon la revendication 1, **caractérisée en ce qu'**elle comprend en outre une huile additionnelle sélectionnée parmi des huiles hydrocarbonées non volatiles d'origine végétale, plus préférablement sélectionnée dans le groupe constitué d'huiles végétales, huiles non volatiles de masse moléculaire élevée comprise entre 350 et 10 000 g/mol, et de mélanges de celles-ci ; plus préférablement sélectionnée parmi de l'huile de canola, huile de graines de ricin commun, triisostéarate de polyglycérol-2, et de mélanges de ceux-ci.

**3.** Composition selon la revendication 1 ou 2, **caractérisée en ce qu'**elle comprend en outre une cire additionnelle choisie parmi des cires hydrocarbonées d'origine végétale ou animale, des esters obtenus par hydrogénation catalytique d'huiles animales ou végétales, ou un mélange de ceux-ci ; de préférence choisie parmi de la cire d'abeille, cire de carnauba, cire de graines d'Helianthus annuus (tournesol), ou un mélange de celles-ci.

**4.** Composition selon l'une quelconque des revendications 1 à 3, **caractérisée en ce qu'**elle comprend au moins une substance colorante choisie parmi des substances colorantes solubles dans l'eau ou insolubles dans l'eau, liposolubles ou non liposolubles, organiques ou minérales, et des matières à effet optique, et de mélanges celles-ci.

**5.** Composition selon l'une quelconque des revendications 1 à 4, **caractérisée en ce que** les triglycérides d'acides gras sont choisis parmi des triglycérides d'acides gras, qui sont notamment en $C_6$-$C_{20}$, de préférence en $C_6$-$C_{14}$, plus préférablement en $C_6$-$C_{12}$, de manière la plus préférée en $C_6$-$C_{10}$ ; de préférence choisis parmi des triglycérides d'acide heptanoïque ou octanoïque, des triglycérides d'acide caprylique/caprique et des mélanges de ceux-ci.

**6.** Composition selon l'une quelconque des revendications 1 à 5, **caractérisée en ce que** le composé pâteux est le Dimère Dilinoléyle Dimère Dilinoléate de Bis-Béhényle/Isostéaryle/Phytostéryle.

**7.** Composition selon l'une quelconque des revendications 1 à 6, **caractérisée en ce que** l'ester linéaire solide est du palmitate de cétyle.

**8.** Composition selon l'une quelconque des revendications 1 à 7, **caractérisée en ce que** ladite au moins une huile choisie parmi des triglycérides d'acides gras est présente selon une quantité allant de 5 % à 40 % en poids, plus préférablement de 8 % à 20 % en poids, par rapport au poids total de la composition.

**9.** Composition selon l'une quelconque des revendications 1 à 8, **caractérisée en ce que** ledit au moins composé choisi parmi un ester de dimère d'acide dilinoléique et polyol(s) ou un ester de ceux-ci est présent selon une quantité allant de 1 % à 40 %. en poids, de préférence de 1 % à 30 % en poids, plus préférablement de 5 % à 20 % en poids, par rapport au poids total de la composition.

**10.** Composition selon l'une quelconque des revendications 1 à 9, **caractérisée en ce que** ladite au moins une cire d'ester linéaire solide dérivé d'acide gras en $C_6$-$C_{30}$ est présente selon une quantité allant de 0,5 % à 20 % en poids, de préférence de 1 % à 10 % en poids, par rapport au poids total de la composition.

**11.** Composition selon l'une quelconque des revendications 1 à 10, dans laquelle le rapport en poids de ladite au moins une huile choisie parmi des triglycérides d'acide gras : ledit au moins un composé pâteux choisi parmi un ester de dimère d'acide dilinoléique et polyol(s) ou un ester de ceux-ci est dans la plage de 1:3 à 3:1, de préférence de 1:2 à 2:1, plus préférablement de 4:5 à 6:5.

**12.** Procédé de soin et/ou de maquillage de matières kératiniques telles que la peau et les lèvres, de préférence les lèvres, par application de la composition telle que définie selon l'une quelconque des revendications 1 à 11 sur les matières kératiniques.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0955039 A
- FR 2792190 A


### Non-patent literature cited in the description


- **C.M. HANSEN.** The three dimensional solubility parameters. *J. Paint Technol.,* 1967, vol. 39, 105